# EUROPEAN PATENT APPLICATION

(11) **EP 4 674 351 A1**
(43) Date of publication of application: **07.01.2026**
(21) Application number: 24186460.2
(22) Date of filing: 04.07.2024
(51) Int. Cl.: A61B 6/03, A61B 6/00

(54) **SCAN CONSISTENCY VERIFICATION**

(71) Applicant: Koninklijke Philips N.V., 5656 AG Eindhoven (NL)
(72) Inventor: SAALBACH, Axel, Eindhoven (NL); HARDER, Tim Philipp, Eindhoven (NL); BUELOW, Thomas, 5656AG Eindhoven (NL); DESHPANDE, Hrishikesh Narayanrao, Eindhoven (NL)
(74) Representative: Philips Intellectual Property & Standards

(57) **Abstract**

A method for monitoring scan consistency in a medical imaging system and a medical imaging system for monitoring scan consistency are provided, as well as a computer program product to carry out the method. The method comprises acquiring a surview image and a scan image of a subject and generating a comparison of the acquired surview image and the scan image. The comparison of the surview image and the scan image is generated based on a deviation criterion. Further, based on the generated comparison a scan consistency level is determined.

## Description

### FIELD OF THE INVENTION

The invention generally relates to monitoring scan consistency in medical imaging. Disclosed are a method for monitoring scan consistency, a medical imaging system, and a computer program product.

### BACKGROUND OF THE INVENTION

Medical imaging systems such as computed tomography (CT) and magnetic resonance imaging (MRI) are used to scan and visualize detailed subject anatomy. These scans are often used to diagnose patients. To achieve a desired scan objective (for example a correct anatomy coverage, or an optimum radiation dose utilization etc.) appropriately planning of such a scan is important. A scan that has been appropriately planned and executed as per the plan leads to a high-quality and efficient acquisition of scan images.

To this end, surview images, also known as localizer radiograph, scanogram, pilot, topogram, survey, pre-scan and scout are acquired prior to the scan for planning purposes. The surview images allow for planning the scan, which is also referred to as the diagnostic scan, according to planning and clinical guidelines. For example, the surview images may provide an overview of the subject's anatomy so that an operator of the imaging system may optimize a field-of-view of a subsequent scan to cover a correct anatomy by taking specific anatomical landmarks or suitable margins into account according to the guideline. Additionally, on the basis of the surview image, the operator may define parameters to be used for imaging during the subsequent scan.

After the acquisition of the surview image, or during the acquisition of the scan image, the subject may move, or there may be breathing motion of the subject, or a cardiac motion of the subject. As a result, a broad range of quality issues could occur including truncated scans, using an inappropriate breathing state for gating, local imaging artifacts due to motion etc. With the high workload at hospitals and imaging centers, it is likely these quality issues are unaccounted for leading to downstream errors such as subject recalls and missed or erroneous diagnoses.

### SUMMARY OF THE INVENTION

It is an insight of the current invention that comparison of the surview scan and the diagnostic scan can provide additional information about the quality of the diagnostic scan. Using this insight, the current invention seeks to provide an approach for monitoring scan consistency in a medical imaging system. This is particularly advantageous when there is subject motion before or during the scanning process including breathing and/or cardiac motion of the subject which can result in artefacts or inconsistencies between scans. The current invention additionally or alternatively seeks to provide an approach for estimating an error or a misalignment of a subject position in a scanner bore of the medical imaging system by monitoring the scan consistency without requiring any additional sensor or hardware.

Thereto a method for monitoring scan consistency in a medical imaging system and a medical imaging system to determine a scan consistency are provided, as well as a computer program product comprising instructions for causing a processor to carry out the method for monitoring scan consistency in the medical imaging system.

The method for monitoring scan consistency in a medical imaging system comprises steps of acquiring a surview image of a subject; acquiring a scan image of the subject; and generating a comparison of the acquired surview image and the scan image based on a deviation criterion. The method also comprises determining a scan consistency level based on the generated comparison. The method is preferably computer-implemented or implemented by other suitable calculation means.

In an embodiment the method comprises displaying a result of the comparison on a user console of the medical imaging system. In this way, an operator of the medical imaging system is able to visualize any inconsistency in the scan on the user console of the medical imaging system and is able to take informed actions.

According to one aspect, the deviation criterion comprises at least one position on the surview image and the scan image. According to a further option, at least one position comprises one or more of a planning box, a landmark, an anatomical structure, a region-of-interest.

According to another aspect, the deviation criterion comprises an image intensity of at least one position of the surview image and the scan image.

According to another aspect, the deviation criterion comprises a size of an anatomy of the subject in the surview image and the scan image. In yet another embodiment, at least an alignment of the subject in a scanner bore of the medical imaging system is estimated based on the generated comparison. In this way, any positioning error or misalignment of the subject in the scanner bore of the medical imaging system can be easily and efficiently determined without requiring any additional sensor or hardware.

According to another embodiment, the method comprises re-acquiring at least a part of the scan image based on the determined scan consistency level. In another option, the subject is positioned in a scanner bore of the medical imaging system based on the generated comparison. In a further embodiment of the method, a scan consistency level is determined again by generating a comparison of the surview image and the re-acquired scan image. This is particularly advantageous as it allows the operator of the medical imaging system to ensure a desired scan consistency and prevent any downstream errors such as patient recalls or erroneous diagnosis.

According to another aspect of the invention, the deviation criterion is a pixel or a voxel level deviation of the acquired surview image and the scan image computed using an optical flow-based technique. In an embodiment the method comprises, generating displacement fields or dense displacement fields on at least the surview image and the scan image.

In a further aspect, deviation criterion is an image level deviation computed by generating a difference image from the acquired surview image and the scan image. According to still another embodiment, the method comprises comparing the surview image with a synthetic surview image generated from a scan image by simulation.

In yet another embodiment of the invention, comparison of the acquired surview image and the scan image based on a deviation criterion comprises applying machine learning or a deep learning-based technique.

In further embodiment, determining a scan consistency level comprises identifying at least one region of the scan image that is affected by motion. This is particularly advantageous when it is required to determine a scan consistency level in presence of subject motion including breathing or cardiac motion occurring during or before the scan.

In yet another embodiment, the method comprises triggering an alert if the scan consistency level is below a predetermined threshold. This is advantageous as the operator is notified beforehand of any inconsistency or quality issues with the scan and appropriate actions can be taken by him/her.

The computer program product comprises instructions that cause a processor to carry out the above-described method when the computer program is executed.

A medical imaging system for determining a scan consistency is provided comprising an imaging device, the imaging device configured to acquire a surview image and a scan image of a subject. Preferably, the medical imaging system comprises a processor, the processor communicates with the imaging device and a user console, the user console communicates with the processor. According to further aspect, the processor is configured to receive the surview image of the subject from the imaging device, and the processor is configured to receive the scan image of the subject from the imaging device. In another aspect, the processor is configured to generate a comparison of the received surview image, and the scan image based on a deviation criterion and determines a scan consistency level based on the generated comparison.

An advantage of the current invention is that by monitoring the scan consistency in a medical imaging system the quality of the scan can be improved and many downstream errors such as patient recalls, erroneous diagnosis can be prevented. An additional advantage is that inconsistencies in the scan due to subject motion including breathing or cardiac motion can be monitored, displayed and based on that appropriate actions can be taken by the operator of the medical imaging system. A further advantage is that the subject can be positioned or aligned in the bore of the medical imaging system accurately and efficiently by monitoring scan consistency without requiring any additional sensors or hardware.

### BRIEF DESCRIPTION OF THE FIGURES

Exemplary embodiments of the invention will now be described with reference to the following Figures, which, unless stated otherwise, are schematic and not to scale, wherein:
Fig. 1 schematically and exemplarity illustrates an imaging system for acquiring CT image data of a subject comprising a system for monitoring scan consistency in a CT imaging device.
Fig. 2 schematically illustrates an example of a computer-implemented method for monitoring scan consistency in an imaging system.
Figs 3a and 3b schematically illustrate a region-of-interest in a surview image and a scan image, respectively.
Figs 3c and 3d schematically illustrate displacement fields computed on an overlay of a surview image and a scan image.
Figs 4a, 4b and 4c schematically illustrate a region-of-interest in a surview image, a scan image, an overlay of surview and scan image, respectively.
Figs 5a and 5b schematically illustrate a surview image and a scan image, respectively.
Figs 5c and 5d schematically illustrate an overlay of a surview image and a scan image.
Fig 6 represents an example of a CT image of lungs of a subject.
Figs 7a, 7b and 7c schematically illustrate projection geometry in a CT imaging system.
Fig. 7d illustrates the width of an anatomy of a subject in image space for different subject table heights.
Figs 8a, 8b and 8c illustrate exemplary embodiments comprising a comparison of images for subject positioning.

### DETAILED DESCRIPTION OF THE INVENTION

In the examples below, medical image data is acquired for a subject. Such a subject can be a human being, particularly a human patient requiring imaging for medical purposes. However, alternative subjects are also envisaged, for example animals such as research animals, pets, or livestock. The approach according to the invention can also be applied to inanimate objects having intrinsic periodic motion that cannot be paused during image acquisition. In the examples, the imaging device is illustrated as a computed tomography (CT) scanner, but other devices may also be used to acquire medical image data.

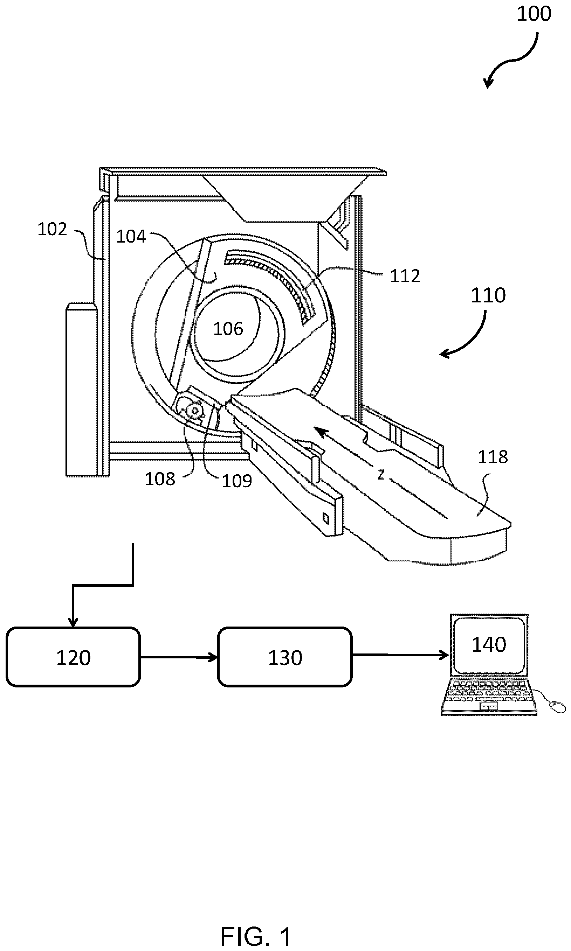

Fig. 1 illustrates an imaging system 100 for acquiring medical image data of a subject.

The imaging system 100 comprises an imaging device 110. The imaging device in this exemplary embodiment is a CT scanner. The computed tomography imaging device 110 includes a stationary gantry 102 and a rotating gantry 104, which is rotatably supported by the stationary gantry 102. When the system is in operation, the rotating gantry 104 rotates around an examination region 106 about a longitudinal or z-axis. A radiation source 108, such as an x-ray tube, is supported by and rotates with the rotating gantry 104 and emits radiation that traverses the examination region 106. A source collimator 109 collimates the emitted radiation to form a generally fan, wedge, or cone shaped radiation that traverses the examination region 106.

A support 118, such as a bed or couch, is provided to support an object or subject to be imaged in the examination region 106. Support 118 is movable along the z-axis in coordination with the rotation of the rotating gantry 104 to facilitate the desired scanning trajectory, preferably a helical scanning trajectory.

A radiation sensitive detector array 112 detects radiation emitted by the radiation source 108 that traverses the examination region 106 and generates projection image data indicative of the detected radiation. The illustrated radiation sensitive detector array 112 includes one or more rows of radiation sensitive photosensor pixels along the z-axis.

In the imaging system 100, an image reconstruction system 120 is provided. The image reconstruction system 120 reconstructs the projection data and generates volumetric image data indicative of the examination region 106, including structure of an object or subject disposed therein. One or more images can be generated from the volumetric image data.

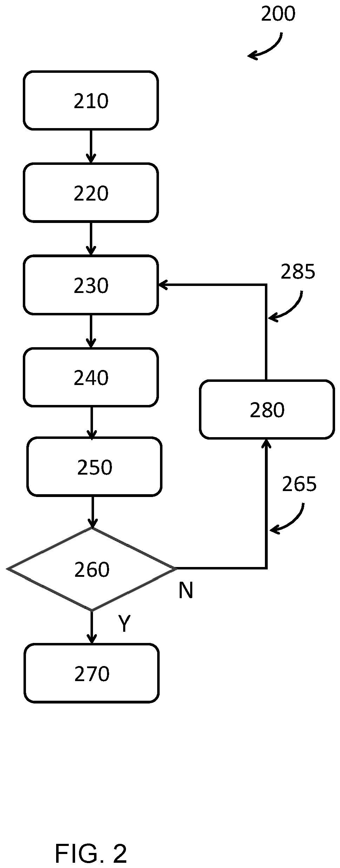

The imaging system 100 further comprises a processor 130 e.g. a computer or other processing device capable of processing image information which will be described in further detail with reference to Fig. 2. In an embodiment, the processor 130 is configured to communicate with the imaging device 110, in this example the CT imaging device. In an embodiment, the processor 130 communicates with the imaging device 110 by receiving the image data from the reconstruction system 120.

The imaging system 100 further comprises a user console 140. The user console 140 is configured to communicate with the processor 130.The user console 140 may include a human readable output device such as a monitor or display and an input device such as a keyboard and mouse. The user console is configured to display one or more generated images.

Reference is now made to Fig. 2, which shows a flow chart of a computer-implemented method 200 for monitoring a scan consistency in a medical imaging system.

The method 200 comprises step 210 for acquiring one or more surview images. The surview image is acquired by an imaging device, for example, from the computed tomography imaging device 110 illustrated in Fig. 1. The surview image may be 2D (two dimensional) projection surview image(s), for example frontal and sagittal, or a 3D (three dimensional) surview image (an image volume). The surview image is preferably acquired with a lower radiation dose than a subsequent scan image (also referred to as a diagnostic image). Since the surview image is acquired at a lower dose, the image contrast and image quality of the surview image may be lower than that of the scan image.

At step 220 one or more scan images are acquired. The scan image may be acquired using the same device used to acquire the surview image or using a different imaging device. Preferably the same device is used. Using the same device will reduce the risk of subject movement between image acquisitions. In a preferred embodiment the scan image is acquired with radiation dose higher than that of the surview image. Hence, the image quality and contrast of the scan image may be higher than that of the surview image. A lower quality image is sufficient for scan planning, while a high-quality image is required for medical diagnosis.

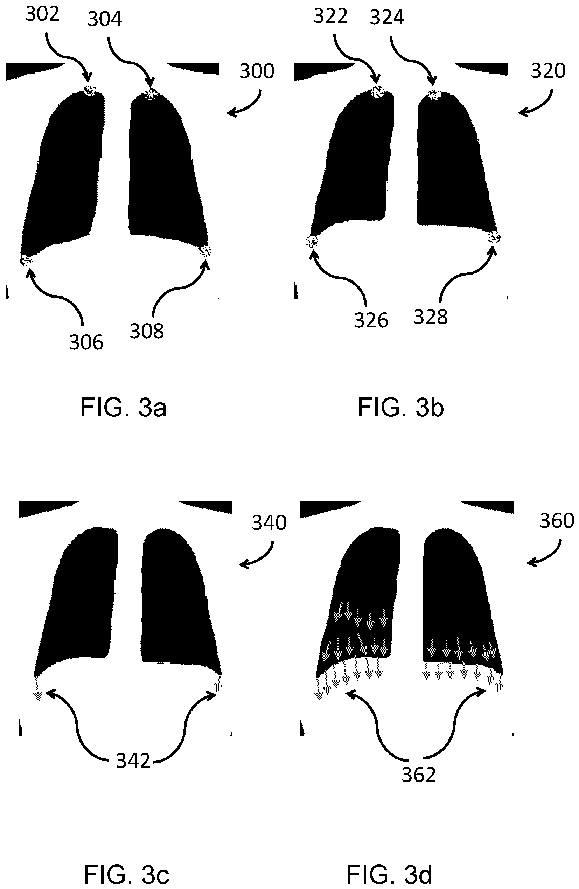

At step 230 a comparison of the one or more acquired surview images and the one or more acquired scan images is generated. The comparison of the images is generated based on a deviation criterion which will be described in more detail with respect to Figs. 3-8.

At step 250 a scan consistency level is determined. The scan consistency level is determined based on the comparison of the scan image with a baseline image, for example, the surview image. The scan consistency level is an indication of the degree of similarity or mismatch between the images. The scan consistency level can be a number or any other visual indicator indicating the degree of mismatch or similarity, but it can also be a two-dimensional image or three-dimensional volume. For example, the scan consistency level can be a color-coded heat map of the comparison or a deformation vector field.

In an exemplary embodiment if two or more scan images are acquired, each of the scan image may be compared to the surview image to determine a consistency level between the two scan images with respect to the surview image i.e. the baseline image. For example, if two scans of the subject are taken at different time points, by comparing each of the two scan images taken at different time points to the surview image a consistency level among the scans may be determined. In an embodiment, if a new scan image is acquired its consistency level with one or more previous scan images that resulted in a high-quality diagnostic image may be determined.

Fig. 2 also shows an optionally additional step 240. At step 240 a result of the comparison of the surview image and the scan image is displayed on a user console of the imaging system, for example user console 140 of imaging system 100 in Fig. 1. The user console may be a display device for displaying one or more images. The result of the comparison is displayed so that the operator of the imaging device is able to visualize a degree of mismatch or similarity, of the surview image and scan image which will be explained further in more detail with respect to Figs. 3-8. In a preferred embodiment, the scan consistency level determined at step 250 may also be displayed on the user console, for example as a number, a bar, or an image, so that the operator is able to take a decision as to whether the current scan image is consistent with one or more previous scan images.

Fig. 2 further shows optionally additional steps 260, 270 and 280. These steps may be implemented additionally or alternatively to optional step 240. At step 260 the scan consistency level is compared to a predetermined threshold. The predetermined threshold may be input by the operator of the imaging system, which may in turn be defined based on a desired scan consistency level required to be attained for a particular scanning session. In an alternate option, the predetermined threshold may be predefined by the processor of the imaging system based on any previous similar scanning sessions, for example, based on similar scanning sessions that resulted in high quality diagnostic images.

If the scan consistency level is above a predetermined threshold, at step 270, the acquisition is completed.

If the scan consistency level is below a predetermined threshold, the method proceeds to step 280 where a whole or a part of the scan image is reacquired. On reacquiring the whole or part of the scan image the method returns to step 230 where a comparison of the surview image and the reacquired scan image is generated, and the method steps 250-260, and optionally 240 are repeated.

The above-mentioned one or more method steps for monitoring scan consistency may be computer-implemented, in an example, by the processor 130 of the imaging system 100.

Reference is now made to Fig. 3, which illustrates schematic diagrams of lung image of the subject.

Figs. 3a and 3b show schematic illustration of a region-of-interest (ROI), for example lungs in this case, in a surview image 300 and scan image 320, respectively. As mentioned earlier, a comparison of the surview image and the scan image is generated based on a deviation criterion. In one embodiment, the deviation criterion may be predefined. In an alternative embodiment, the deviation criterion may be input on the user console before or after the acquisition of the surview image. This provides an advantage that an operator may be able to input an appropriate deviation criterion based on which he/she would like the surview image and scan image to be compared. In another alternate embodiment, the deviation criterion may be identified automatically by the imaging system, for example, by a processor of the imaging system, after the acquisition of the surview image. In another embodiment deviation criterion may be input by the operator or identified by the processor after the acquisition of the scan image.

In one possible embodiment the deviation criterion is a position on the surview image and the scan image, preferably a position of one or more anatomical landmarks identified in the surview image and the scan image. In another optional embodiment deviation criteria may be one or more planning boxes defined by the operator in the surview image. More specifically, the deviation criterion may be a position of one or more planning boxes in the surview image and the scan image. The planning box may indicate a region-of-interest to be scanned or the planning box may indicate a recognized anatomical landmark identified by the operator in the surview image. The deviation criterion as the position of one or more anatomical landmarks may be selected automatically, for example, if one or more anatomical landmarks are identified in the surview image and/or scan image by the processor of the imaging system. In another example, the deviation criterion may be selected automatically by the processor based on one or more previous scans.

The schematic illustration in Fig. 3a shows surview image 300 with anatomical landmarks 302, 304, 306, 308 identified. The identification of anatomical landmarks is accomplished by any suitable methods known in the art including but not limited to machine learning e.g. a deep learning-based technique, an atlas-based registration technique, a heat-map based technique, an optical flow-based technique, computer vision techniques e.g. by using a sliding window classifier or any combination of the methods. Similarly, Fig. 3b depicts a ROI of the subject, lungs in this case, in a scan image 320. The anatomical landmarks 322, 324, 326, 328 in the scan image 320 may be identified by any suitable methods as described above. Identified anatomical landmark 322 in scan image 320 corresponds to the landmark 302 in surview image 300, landmark 324 to 304, landmark 326 to 306, and landmark 328 to 308. Fig. 3c and Fig. 3d depict a schematic diagram of an overlay image 340, 360 of the surview image 300 and the scan image 320. Figs. 3c and 3d shows an exemplary embodiment where the surview image and the scan image are overlayed and a comparison of the surview image and the scan image is generated. However, it may be possible to generate a comparison of the surview image 300 and the scan image 320 without overlaying the two images. In one option, the comparison of the surview image and the scan image is generated based on a position of identified one or more anatomical landmarks in the surview image and the scan image. In another option, a comparison of the surview image and scan image is generated based on position of one or more planning boxes in the surview and scan image. For example, the position of the anatomical landmarks 302, 304, 306, 308 in the surview image 300 may change or get displaced to the corresponding landmarks 322, 324, 326, 328 in the scan image 320. The change in position of the anatomical landmark or planning box may be displayed or highlighted on the user console of the imaging system.

In yet another embodiment of the invention one or more anatomical structures are identified in the surview image and the scan image. Thereafter, a comparison of the surview image and the scan image is generated based on position of the identified one or more anatomical structure in the surview image and scan image. The comparison may be generated by the method described above.

The change in position of one or more landmarks, one or more planning box or one or more anatomical structures in the surview and scan image may be directly visualized by the operator on the user console of the imaging system. Alternatively, the change in position of one or more anatomical landmarks, planning box or one or more anatomical structure in the surview image and the scan image may be computed by any suitable means and visualized on the user console.

In an embodiment the change in the position is visualized by an optical flow method that computes displacement field of the identified landmark or anatomical structure in the surview and scan image. For example, Fig. 3c depicts sparse displacement field 342 computed on an overlay of the surview image and the scan image. In another embodiment as shown in Fig. 3d dense displacement field 362 are computed on an overlay of the surview image and the scan image. Therefore, by computing displacement fields or dense displacement fields the operator may be able to visualize a result of the comparison of the surview image and the scan image, in this embodiment a change in position of the one or more anatomical landmarks or one or more anatomical structures.

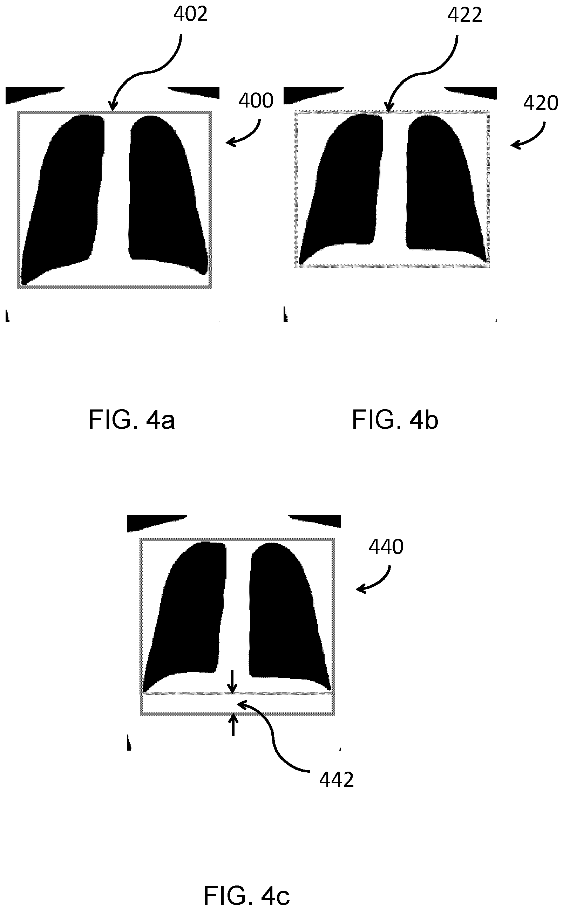

Fig. 4 is another exemplary embodiment of the invention with schematic illustrations of a region-of-interest (ROI) of the subject, lung in this case.

As illustrated in Fig. 4a, in surview image 400, a ROI 402 such as an anatomy of interest, for example lungs in this case, is identified. In this illustrated embodiment of the invention, the deviation criterion is the position of the ROI in the surview and the scan image. The identification of the ROI may be performed manually such as the operator selecting a ROI in a displayed surview image. In an alternate embodiment, the identification of ROI is performed automatically, for example, by a processor or other suitable means. The automatic identification of the ROI may be achieved by any suitable method known in the art, for example, by registration-based techniques, machine learning e.g. deep learning-based techniques or convolution neural network, or computer vision techniques e.g. using a sliding window classifier. The registration technique in an embodiment may be an atlas-based registration technique. The identified ROI, in an exemplary embodiment, is an extent of the anatomy of interest, for example, extent of lungs visible in the image. The ROI 402 in the surview image 400 is identified and displayed to the operator as described above. As illustrated in Fig. 4b a scan image 420 is acquired subsequently to the acquisition of the surview image 400. Further, the corresponding ROI 422 in the scan image 420 is identified and displayed by any suitable method as described above. The surview image, the scan image, and the surview image and/or scan image together with the identified ROI may be stored suitably, for example in a memory of the imaging system 100 or in a database for medical images. A comparison of the surview image and the scan image is generated by determining a change between the ROI in the surview image and the corresponding ROI in the scan image. This change may be computed by the processor or any other suitable means. In an embodiment as illustrated in Fig. 4c the change in ROI 442 is computed on an overlay 440 of surview image and scan image. However, a change in the ROI may be computed without overlaying the surview and the scan image. The result of the comparison, for example, change in ROI 442, is displayed and/or highlighted to the operator. In an embodiment of the invention, if the change in ROI is above a predetermined threshold, an alert is triggered to the operator. The alert may prompt the operator to re-acquire a part or whole of the scan image. In an embodiment the re-acquisition of the scan image may be performed with a change in positioning of the subject in a scanner bore of the imaging system.

In another embodiment of the invention the deviation criterion may be a contour of an anatomy of interest. For example, a contour of the anatomy may be identified in the surview image by any suitable methods described above. The corresponding contour is identified in the subsequent scan image and a comparison of the contours in the surview image, and the scan image is generated.

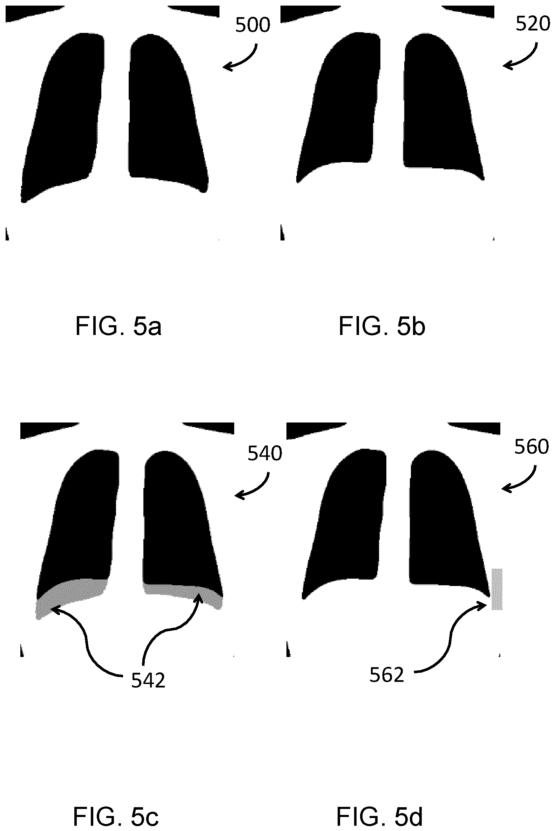

Reference is now made to Fig. 5, which shows schematic diagrams of lung images in another exemplary embodiment of the invention. In Fig. 5a, image 500 illustrates a surview image of a ROI, in this example, lungs of the subject and image 520 in Fig. 5b illustrates a scan image of the lungs of the subject. Preferably, the surview image 500 and the scan image 520 are overlayed such as overlay images 540 and 560. As described above, a comparison of the surview image and the scan image is generated based on a deviation criterion. In this embodiment the deviation criterion is an image level deviation computed by generating a difference image from the surview image and the scan image. In the overlay images 540 and 560 a difference image between the surview image and the scan image is computed. The difference image computed on the overlay images 540 and 560 assists the operator to visualize differences in the surview and the scan image. However, a difference image may be computed without overlaying the surview and the scan image. In an embodiment difference image is computed by calculating a pixel or a voxel level difference, for example, by calculating a cumulative pixel or voxel difference between the surview and the scan image. In an embodiment the pixel or voxel level difference may also be computed using an optical flow-based technique, for example, by generating displacement or dense displacement fields on the surview image and the scan image as already described with reference to Fig. 3c and 3d. Fig. 5c shows a difference image between surview and scan image and with regions 542 showing areas where the cumulative pixel difference between surview image and scan image is higher than a threshold. In an embodiment, regions 542 may be regions affected by motion of the subject. For example, the motion may be due to bodily movement of the subject, a breathing motion of the subject or a cardiac motion of the subject. Thus, region 542 may indicate a blurred region such as an artefact region in the scan image that has been affected by subject motion. In another embodiment Fig. 5d shows an overlay image 560 with an image quality bar 562 that indicates an extent of difference between the surview and the scan image. The image quality bar may show a degree of mismatch between surview and scan image, for example, a degree of blurring or artefact as a result of subject motion. In an example, the length of the quality bar 562 may indicate to the operator the degree of mismatch between the surview image and scan image and trigger an alert to the operator on the user console if the degree of mismatch is above a predetermined threshold.

In another exemplary embodiment of the invention, the deviation criterion is an image intensity of one or more positions of the surview image and the scan image. A comparison of the surview image and the scan image is generated based on the image intensity of one or more positions of the surview and scan image. The image intensity may be computed of each pixel or voxel in the surview and the scan image. In an example, intensity difference is computed of each pixel or voxel and a cumulative intensity difference of a region of interest is displayed on the user console of the imaging system to highlight a mismatch between the images. In another example, instead of computing an intensity difference, a ratio or a statistical analysis of the intensity of the pixel or voxel is performed and the result displayed to highlight a mismatch between the images.

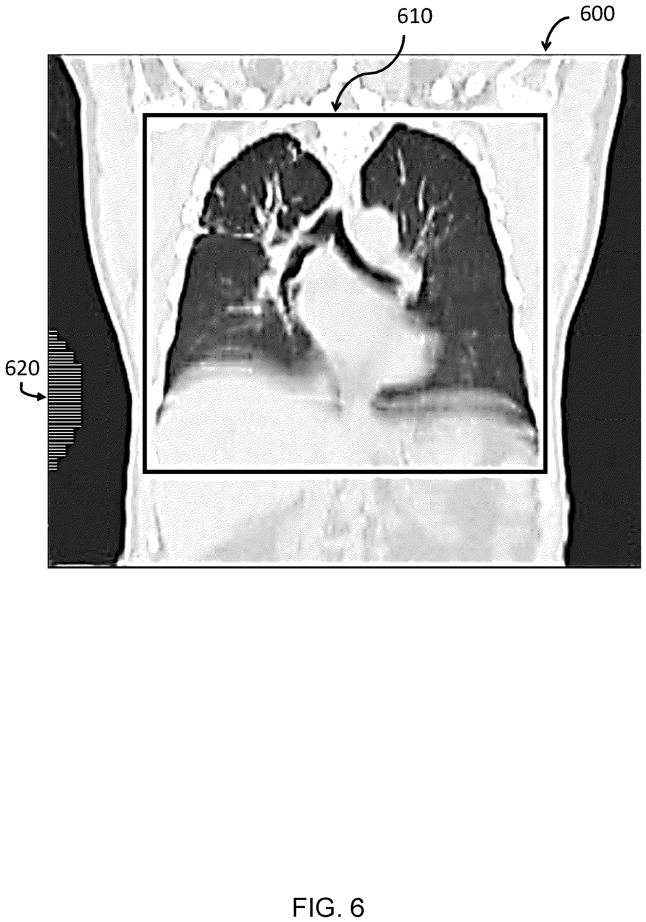

Fig. 6 represents an example of a computed tomography image of lungs of a subject. As illustrated in the Figure, box 610 depicts a ROI in the lung image 600. A difference map or difference image is generated for the ROI 610 by comparing the ROI 601 with a ROI of a surview image using any suitable method as described above. The difference map may be generated by computing a pixel level difference between the ROI of the scan image and corresponding ROI of the surview image. The difference map may be visualized as a heat map to highlight areas with mismatch between the image 600 and surview image. The mismatch may be due to a motion of the subject as explained earlier. In an embodiment the difference map is visualized in the form of motion bars 620 in the image 600. The amount or severity of motion may be encoded as length of motion bars. For example, severe motion may be indicated by a greater length of motion bars and minor motion may be indicated by smaller length of motion bars. If motion bars indicate severe motion an alert may be triggered to the operator and enables the operator to identify regions or slices in the image that is affected by motion and take corrective action, for example, plan for reacquisition of the image or slice. This is particularly advantageous as it improves clinical workflow by enabling the operator to take corrective actions earlier and prevent any downstream errors such as subject recalls and leads to improved subject diagnosis.

Hence, according to the embodiments of the invention a comparison of the surview image and the scan image is generated and visualized, the comparison is based on appropriate deviation criterion(s), which is either defined by the operator of the imaging system or generated automatically by the imaging system based on current or previous scans. For the scan image, a scan consistency level is determined based on the generated comparison. The scan consistency level is a degree of mismatch or similarity between the scan image and the surview image. In another embodiment the scan consistency level is a degree of mismatch or similarity between the scan image and one or more previous scan images taken at different time points for which comparison has already been generated with the same or a different surview image. Hence, the scan consistency level may also be determined from a previously generated comparison of a scan and surview image. The degree of mismatch or similarity indicates for example, an extent of displacement of anatomical landmark(s) in the scan image, an extent of displacement of a region-of-interest or a planning box in the scan image. In other embodiments it may indicate an extent of variation of difference image, an extent of variation of intensity difference of a region-of-interest. The extent of variation of difference image or intensity difference may indicate for example, an amount of blurring or artefact due to subject motion including breathing or cardiac motion of the subject. This may also enable determination of regions of the scan image that are affected by motion, including subject motion. In exemplary embodiments the scan consistency level may be a number or a visual indicator indicating the degree of mismatch or similarity, but it can also be a two-dimensional image or three-dimensional volume. For example, the scan consistency level can be a color-coded heat map of the comparison or a deformation vector field. This determined consistency level of the scan image may be stored in a database or memory of the imaging system with or without the scan image. If a new scan session is initiated for the same subject, and another comparison of the surview and scan image is generated, a consistency level of the current scan image with one or more previous scan image can be determined. The previous scan images may be the ones where a comparison with the surview image has been performed and satisfactory image quality has been obtained. If a scan image is highly inconsistent with previous scan image, this may be an indication of an artefact, subject motion, or the scan image may not be suitable for high quality diagnosis. Therefore, the invention has advantage that it provides the operator with an indication of scan quality and allows him/her to take appropriate measures such as re-acquiring whole or part of the scan image and thereby preventing any downstream errors such as subject recalls and erroneous diagnosis.

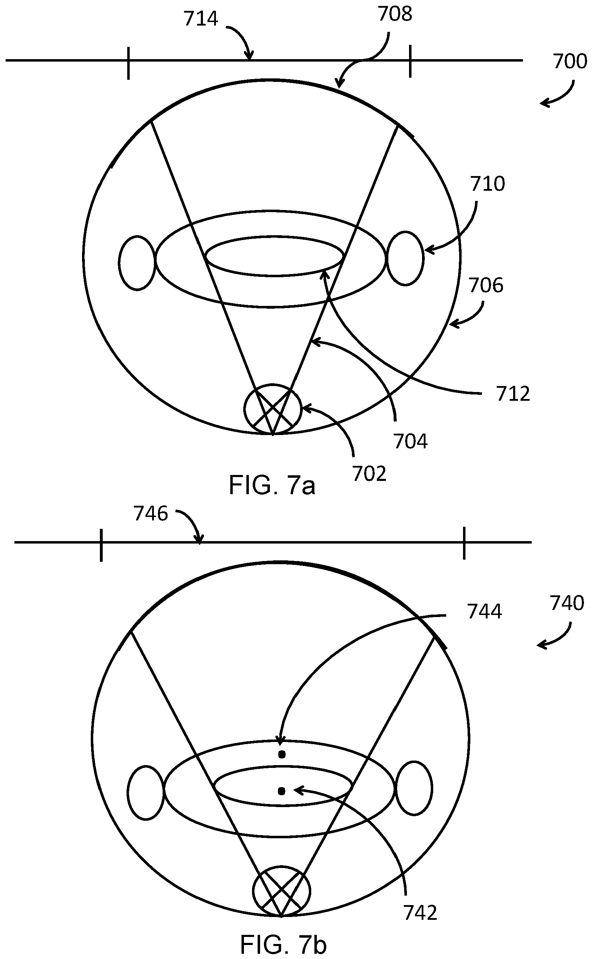

Reference is now made to Figs. 7a to 7c which illustrate schematic diagrams to compare surview images and scan images for estimating an alignment of the subject in the scanner bore of the imaging system.

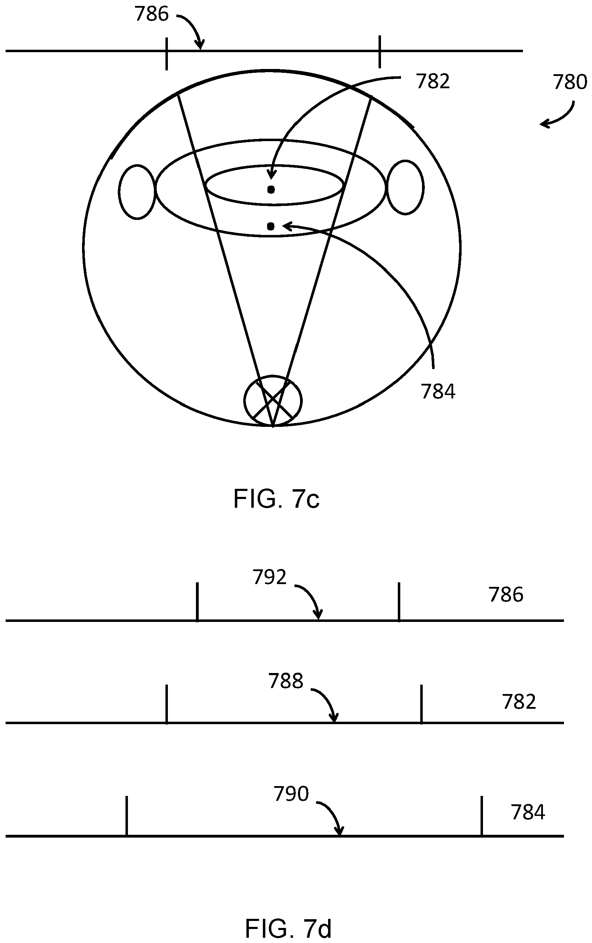

In an embodiment of the invention, a deviation criterion comprises a size of an anatomy of the subject in the surview image and scan image. A comparison of the surview image and the scan image is generated based on the deviation criterion. This is explained in more detail in Fig. 7. Turning first to Fig. 7a, which schematically illustrates a projection geometry 700 for comparing a surview image with a 3D clinical scan image when subject isocenter is aligned with the isocenter of the imaging device, for example a CT scanner. Specifically, a comparison is made of 2D surview image with a simulated surview image that is generated from 3D scan, for example by projection or simulation. The 2D surview image is acquired as a projection image with X-ray source in a fixed position. The comparison of the surview and simulated surview image is used to position a subject in the scanner bore of an imaging system, such as a computed tomography imaging system as will be described below. Referring to Fig. 7a, an X-ray source 702 emits X-ray beam 704 towards a target anatomy 712 of a subject 710, the subject being placed in gantry 706 of the imaging device. In an embodiment a target anatomy may be lungs of the subject. The detector 708 receives an attenuated X-ray beam that passes through the subject and an image of the anatomy is reconstructed. In the embodiment of Fig. 7a, 714 represents the width of the anatomy of the subject in image space when the scanner isocenter and the subject center are aligned. The width of the anatomy in image space is an apparent width of the anatomy, for example, width with respect to a viewing condition such as a window width or a window center setting in a CT scanner as known in the art. Referring now to Fig. 7b which shows a schematic projection geometry when scanner isocenter 744 is above the subject center 742. Such cases may be possible when the table in which the subject is positioned is much lower than desired for obtaining an optimum image. In this case the width of the anatomy in image space, for example the apparent width of lungs, 746 is obtained. The obtained apparent width of anatomy 746 is larger as compared to the apparent width of anatomy 714 when the scanner isocenter and the subject isocenter are aligned. Now, referring to Fig. 7c, 780 is a schematic diagram of a projection geometry when a scanner isocenter 784 is below the subject center 782. Such cases may be possible when the when the table in which the subject is positioned is much higher than desired for obtaining an optimum image. In this case the obtained apparent width of the anatomy 786 is smaller as compared to the apparent width of the anatomy 714 when the scanner isocenter and the subject isocenter are aligned. Fig. 7d shows a comparison of the apparent width of the anatomy 788, 790 and 792 in image space for a correct table height, a lower table heigh and a higher table height, respectively. Therefore, the apparent width of the anatomy of the subject in image space may be an indicator as to a correct table height of the subject. Thus, the comparison of an apparent size of an anatomy for different subject heights may assist an operator in positioning or aligning the subject to a correct height in the scanner for obtaining an optimal image.

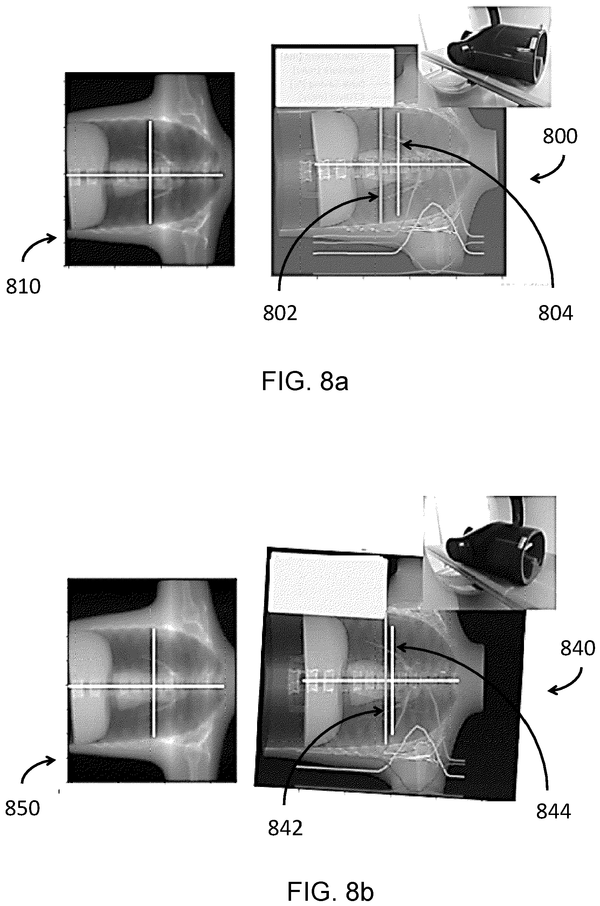

Figs. 8a to 8c further illustrate exemplary embodiments comprising comparison of images for subject positioning.

Fig. 8a shows a comparison of a scan image 800 and a surview image 810 for a correct subject table height. In a particular embodiment of the invention, a simulated surview image may be used as the surview image and the acquired surview image is used as the scan image. The simulated surview image is generated as a reference from a scan image taken at the desired correct table height by simulation. A particular advantage of this approach is that the table height may be corrected before a diagnostic scan is acquired at a higher dose. In this way acquisition of a second, corrected diagnostic scan may be avoided.

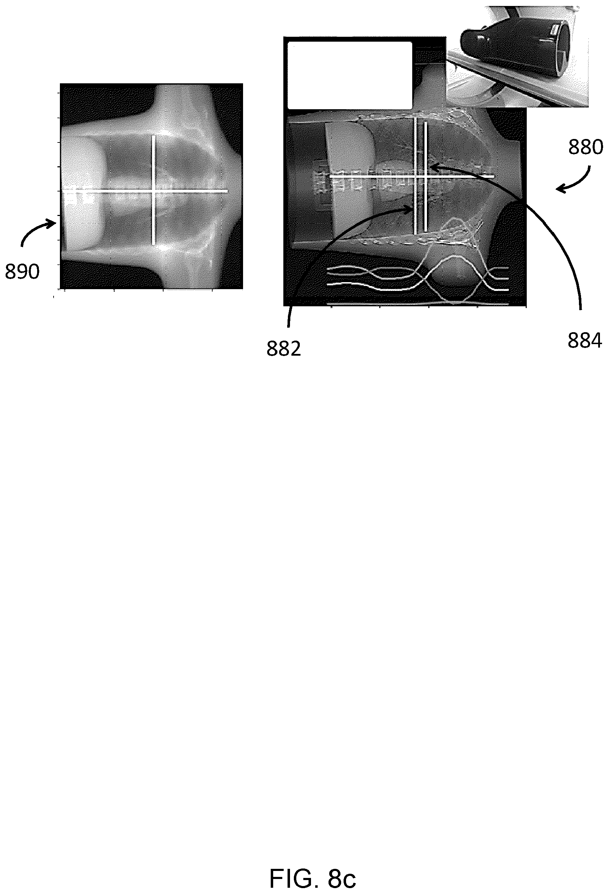

The comparison of the scan image and surview image is generated based on a deviation criterion, in an exemplary embodiment deviation criterion is the size of an anatomy of interest of the subject. The anatomy of interest in Fig. 8 are the lungs, and an apparent lung width in the images may be determined, for example, by using a segmentation algorithm or any other method known in the art. The apparent width of the lungs, is for example, width with respect to a viewing condition such as a window width or a window center setting in a CT scanner as known in the art. In Fig. 8a lines represented by numerals 802 and 804 shows apparent lung width in the surview image and the simulated surview image respectively. Now, Fig. 8b shows surview image 840 and simulated surview image 850 for a low subject table height. The surview image 840 is used as a scan image and simulated surview image 850 is used as a surview image as mentioned above. The lines represented by numerals 842 and 844 on the surview image 840 shows apparent lung width in surview image 840 and simulated surview image 850 respectively. Turning now to Fig. 8c which shows a surview image 880 and a simulated surview image 890 for a high table height. The lines represented by numerals 882, 884 shows apparent lung width in surview image 880 and simulated surview image 890 respectively. As evident from Figs. 8a-8c, the lower the subject table height, the larger the apparent width of the lungs. In this example, the length of 842 is largest and that of 882 is smallest when the lengths of 802, 842 and 882 are compared against each other. Therefore, the apparent width of the anatomy, for example the apparent width of the lungs in this case, is a deviation criterion for comparing the surview image and the scan image. This comparison may be stored in a memory or a database of the imaging system. The memory or database of the imaging system may store the apparent width of the lungs for a correct subject table height and the corresponding scan and surview image. In an embodiment, the apparent width of the lungs and the corresponding image may be stored for one or more table heights. The table heights may correspond to scan image which resulted in high quality diagnostic scan. Any subsequent scan image that is acquired an apparent lung width for that scan image is determined and is compared to the one or more stored apparent lung width. In this way a consistency level may be determined based on comparing the scan image and the surview image using apparent width of the lungs as deviation criterion. The consistency level may indicate, for example, an extent of variation of the lung width in the scan image and may be visualized by any suitable means as already described above. The determination of consistency level is advantageous since the operator is able to take immediate actions e.g. adjusting the table height in this case and thereby prevent any errors which may affect the quality of the acquired scan.

Any of the method steps disclosed herein may be recorded in the form of a computer program comprising instructions which when executed on a processor cause the processor to carry out such method steps. The instructions may be stored on a computer program product. The computer program product may be provided by dedicated hardware as well as hardware capable of executing software in association with appropriate software. When provided by a processor, the functions can be provided by a single dedicated processor, by a single shared processor, or by a plurality of individual processors, some of which can be shared. Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid-state memory, magnetic tape, a removable computer diskette, a random-access memory "RAM", a read-only memory "ROM", a rigid magnetic disk and an optical disk. Current examples of optical disks include compact disk - read only memory "CD-ROM", compact disk - read/write "CD-R/W", Blu-Ray^{™} and DVD. Examples of a propagation medium are the Internet or other wired or wireless telecommunication systems.

Variations to the disclosed embodiments can be understood and effected by those skilled in the art in practicing the claimed invention, from a study of the drawings, the disclosure, and the appended claims. It is noted that the various embodiments may be combined to achieve further advantageous effects.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A method for monitoring scan consistency in a medical imaging system, comprising the steps of:
- acquiring a surview image of a subject;
- acquiring a scan image of the subject;
- generating a comparison of the acquired surview image and the scan image based on a deviation criterion;
- determining a scan consistency level based on the generated comparison.

2. The method according to claim 1, further comprising displaying a result of the comparison on a user console of the medical imaging system.

3. The method according to claim 1, wherein the deviation criterion comprises at least one position on the surview image and the scan image.

4. The method according to claim 3, wherein the at least one position comprises one or more of:
- a planning box,
- a landmark,
- an anatomical structure,
- a region-of-interest.

5. The method according to claim 1, wherein the deviation criterion comprises an image intensity of at least one position of the surview image and the scan image.

6. The method according to claim 1, wherein the deviation criterion comprises a size of an anatomy of the subject in the surview image and the scan image.

7. The method according to claims 1, 6 wherein at least an alignment of the subject in a scanner bore of the medical imaging system is estimated based on the generated comparison.

8. The method according to claim 1, wherein based on the determined scan consistency level at least part of the scan image is re-acquired.

9. The method according to claim 8, wherein a scan consistency level is determined again by generating a comparison of the surview image and the re-acquired scan image.

10. The method according to claim 1, wherein the deviation criterion is a pixel or a voxel level deviation of the acquired surview image and the scan image computed using an optical flow-based technique.

11. The method according to claim 1, wherein the deviation criterion is an image level deviation computed by generating a difference image from the acquired surview image and the scan image.

12. The method according to claim 1, wherein determining a scan consistency level comprises identifying at least one region of the scan image that is affected by motion.

13. The method according to claims 1, 12 further comprising triggering an alert if the scan consistency level is below a predetermined threshold.

14. A medical imaging system, comprising:
- an imaging device, the imaging device configured to acquire a surview image and a scan image of a subject;
- a processor, the processor communicating with the imaging device; and
- a user console, the user console communicating with the processor;
wherein the processor is configured to perform the steps of:
receiving the surview image of the subject from the imaging device;
receiving the scan image of the subject from the imaging device;
generating a comparison of the received surview image and the scan image based on a deviation criterion;
determining a scan consistency level based on the generated comparison.

15. A computer program product comprising instructions for causing a processor to carry out the method according to any of claims 1-13, when the computer program is executed.
